# EUROPÄISCHE PATENTANMELDUNG

(11) **EP 1 394 184 A1**
(43) Veröffentlichungstag der Anmeldung: **03.03.2004**
(21) Anmeldenummer: 03026898.1
(22) Anmeldetag: 16.02.1995
(51) Int. Cl.: C07K 19/00, C12N 15/62, A61K 38/17, A61K 39/395, A61P 31/18, A61P 37/00

(54) **Hemmer von Apoptose**

(30) Priorität: 08.04.1994 DE 4412177
(62) Teilanmeldung aus: 95909730.4
(71) Anmelder: Deutsches Krebsforschungszentrum Stiftung des öffentlichen Rechts, 69009 Heidelberg (DE)
(72) Erfinder: Die Erfindernennung liegt noch nicht vor
(74) Vertreter: Weiss, Wolfgang, Dipl.-Chem. Dr.

(57) **Zusammenfassung**

Die Erfindung betrifft Mittel zur Hemmung von Apoptose. Ferner betrifft die Erfindung Verbindungen, die sich zur Hemmung von Apoptose eignen, sowie Verfahren zu deren Herstellung.

## Beschreibung

Die Erfindung betrifft Mittel zur Hemmung von Apoptose. Ferner betrifft die Erfindung Verbindungen, die sich zur Hemmung von Apoptose eignen, sowie Verfahren zu deren Herstellung.

Apoptose ist die Bezeichnung für programmierten Zelltod. Dieser findet sich, z.B. bei der Organentwicklung und Metamorphose, der Gewebeatrophie und Tumorregression. Apoptose ist mit einer Kondensation des Cytoplasmas, einem Verlust von Plasmamembranvilli, einer Segmentation des Kems und extensivem Abbau chromosomaler DNA verbunden (vgl. Oehm, A. et al., The Journal of Biological Chemistry, Band 267, Nr. 15 (1992), Seiten 10709-10715).

In Zellen, die für Apoptose positiv sind, findet sich oftmals ein mit APO-1 bezeichnetes Zell-Oberflächenprotein. Dieses Glycoprotein wird der Tumornekrosefaktor/Nervenwachstumsfaktor-Rezeptor-Famifie zugerechnet. Durch Crosslinking von APO-1 über einen anti-APO-1-Antikörper wird Apoptose bei den genannten Zellen induziert (vgl. Oehm, A. et al., supra). Gleiches scheint auch durch Bindung eines mit APO-1-Ligand bezeichneten löslichen oder Membran-gebundenen Proteins an APO-1 bewirkt zu werden (vgl. Suda, T. und Nagata, S., J. Exp. Med., The Rockefeller University Press, Band 179 (1994), Seiten 873-879).

Über die Hemmung von Apoptose ist dagegen nichts bekannt. Dies wäre aber umso notwendiger, da jüngste Arbeiten des Anmelders zeigen, daß Apoptose auch bei verschiedenen Erkrankungen, wie AIDS und Autoimmunerkrankungen, auftritt. Bei AIDS scheint Apoptose für die starke Abnahme der CD4-T Zellen verantwortlich zu sein.

Der vorliegenden Erfindung liegt somit die Aufgabe zugrunde, ein Mittel bereitzustellen, mit dem Apoptose gehemmt werden kann.

Erfindungsgemäß wird dies durch Bereitstellung eines Mittels erreicht, das sich dadurch auszeichnet, daß es eine bis alle Komponenten enthält von:
(a) eine APO-1 hemmende Verbindung,
(b) eine den APO-1-Ligand hemmende bzw. abfangende Verbindung, und
(c) eine den intrazellulären APO-1-Signalweg hemmende Verbindung, wobei die Komponente(n) in einem Individuum nicht als fremd angesehen wird (werden), sowie übliche Hilfsstoffe.

Der Ausdruck "APO-1 hemmende Verbindung" umfaßt jegliche zur Hemmung von APO-1 geeignete Verbindung. Vorzugsweise ist dies ein blockierender, nichtcytotoxischer anti-APO-1-Antikörper oder ein anti-APO-1-Antikörper ohne Fc-Teil, z.B. ein F(ab)-, F(ab)₂- oder Fᵥ-Fragment eines anti-APO-1-Antikörpers. Die Hersiellung eines solchen Fragments erfolgt in üblicher Weise, wobei der Fachmann z.B. von dem in Oehm et al., supra beschriebenen anti-APO-1-Antikörper oder von dem in Dhein, J. et al., The Journal of Immunology, Band 149, Nr. 10, (1992), Seiten 3166-3173 beschriebenen anti-APO-1-F(ab)₂-Fragment ausgeht. Letzteres kann auch direkt eingesetzt werden.

Das Fehlen des Fc-Teils in vorstehendem Antikörper verhindert das Crosslinking von gebundenem APO-1 und somit die Induktion von Apoptose. Überraschenderweise wird APO-1 durch einen solchen Antikörper gehemmt.

Als weitere bevorzugte "APO-1 hemmende Verbindung" ist ein APO-1-Ligand-Analogon zu nennen. Ein solches bindet nach wie vor an APO-1, induziert jedoch nicht mehr den intrazellulären APO-1-Signalweg. Die Herstellung eines solchen Analogons erfolgt in üblicher Weise, wobei der Fachmann z.B. von dem in Suda, T. und Nagata, S., supra beschriebenen APO-1-Ligand ausgeht.

Der Ausdruck "APO-1-Ligand hemmende bzw. abfangende Verbindung" umfaßt jegliche zur Hemmung bzw. zum Abfangen des APO-1-Ligand geeignete Verbindung. Vorzugsweise ist dies eine der folgenden Verbindungen:
- ein anti-APO-1-Ligand-Antikörper,
- ein APO-1,
- eine extrazelluläre APO-1-Domäne,
- eine Verbindung mit mindestens einer extrazellulären APO-1-Domäne und einem Träger,
- ein eine APO-1-Ligand-Bindungsstelle aufweisendes Peptid, und
- eine Verbindung mit mindestens einem eine APO-1-Ligand-Bindungsstelle aufweisenden Peptid und einem Träger.

Die Herstellung einer solchen Verbindung erfolgt in üblicher Weise. Der Fachmann wird zur Herstellung eines anti-APO-1-Ligand-Antikörpers z.B. von dem in Suda, T. und Nagata, S., supra beschriebenen APO-1-Ligand ausgehen. Er wird darauf achten, daß der Fc-Teil des Antikörpers in einem Individuum nicht als fremd angesehen wird. Verfahren, die dies ermöglichen, sind ihm bekannt. Ferner wird der Fachmann hinsichtlich der Herstellung von APO-1 bzw. einer extrazellulären APO-1-Domäne z.B. von dem in der EP-92 107 060.3 beschriebenen APO-1 bzw. seiner extrazellulären Domäne ausgehen. Desweiteren wird er zur Herstellung eines eine APO-1-Ligand-Bindungsstelle aufweisenden Peptids z.B. die Kombination aus vorstehendem APO-1-Ligand und vorstehendem APO-1 bzw. seiner extrazellulären Domäne nutzen.

Die Herstellung einer Verbindung mit mindestens einer extrazellulären APO-1-Domäne und einem Träger wird nachstehend beispielhaft beschrieben. In analoger Weise kann eine Verbindung mit mindestens einem eine APO-1-Ligand-Bindungsstelle aufweisenden Peptid und einem Träger hergestellt werden.

Der Ausdruck "eine den intrazellulären APO-1-Signalweg hemmende Verbindung" umfaßt jegliche zur Hemmung des intrazellulären APO-1-Signalwegs geeignete Verbindung. Vorzugsweise ist dies eine der folgenden Verbindungen:
- ein Hemmer des "interleukin-1β converting enzyme" (ICE), insbesondere 3,4-Dichlorisocoumarin (DCI), YVAD-CHO, ein ICE-spezifisches Tetrapeptid, oder CrmA, ein Protein von Vaccinia-Virus bzw. Derivate davon. Günstig kann es sein, YVAD-CHO oder CrmA bzw. Derivate davon als exprimierbare Nukleinsäuren zu verwenden. Ferner kann es günstig sein, eine ICE-anti-Sinn Nukleinsäure bzw. ein Derivat davon als ICE-Hemmer zu verwenden.
- ein Hemmer von ICE Struktur-verwandten Proteasen, insbesondere von Nedd-2/ich-1 oder priCE.

Die Herstellung einer solchen Hemmer-Verbindung erfolgt in üblicher Weise. Der Fachmann wird zur Herstellung von DCI bzw. einem Derivat davon z.B. auf die Arbeit von Harper, J.W. et al., Biochemistry, 24, 1831 - 1841, (1985) zurückgreifen. Ferner wird er hinsichtlich YVAD-CHO bzw. eines Derivats davon z.B. die Arbeit von Thornberry, N.A. et al., Nature 356, 768 - 774, (1992) heranziehen. Desweiteren wird er bezüglich CrmA oder eines Derivats davon z.B. auf die Arbeit von Ray, C.A. et al., Cell 69, 597 - 604, (1994) zurückgreifen. Darüber hinaus wird er hinsichtlich der ICE-Anti-Sinn-Nukleinsäure bzw. eines Derivats davon z.B. die Arbeit von Cerretti et al., Science 256, 97 - 100, (1992) heranziehen. Im weiteren wird er bezüglich der Proteasen Nedd-2/Ich-1 bzw. priCE z.B. auf die Arbeit von Wang, L. et al., Cell 78, 739 - 750, (1994) bzw. von Lazebnik, Y. A. et al., Nature 371, 346 - 347, (1994) zurückgreifen.

Erfindungsgemäß wird vorstehendes Mittel besonders zur Hemmung von Apoptose bei einer mit einer HIV-Infektion assoziierten Erkrankung eingesetzt. Als besonders vorteilhaft erweist sich das Mittel dabei, wenn es ferner eine bis alle Komponenten enthält von:
(a) eine den TAT-Rezeptor hemmende Verbindung,
(b) eine TAT hemmende bzw. abfangende Verbindung,
(c) eine den intrazellulären TAT-Rezeptor-Signalweg hemmende Verbindung,
(d) eine den CD4-Rezeptor hemmende Verbindung,
(e) eine gp120 hemmende bzw. abfangende Verbindung, und
(f) eine den intrazellulären CD4-Rezeptor-Signalweg hemmende Verbindung, wobei die Komponente(n) in einem Individuum nicht als fremd angesehen wird(werden).

Der Ausdruck "eine den TAT-Rezeptor hemmende Verbindung" umfaßt jegliche zur Hemmung des TAT-Rezeptors geeignete Verbindung. Vorzugsweise ist dies ein anti-TAT-Rezeptor-Antikörper. Die Herstellung eines solchen erfolgt in üblicher Weise. Der Fachmann wird darauf achten, daß der Fc-Teil des Antikörpers in einem Individuum nicht als fremd angesehen wird. Verfahren, die dies ermöglichen, sind dem Fachmann bekannt.

Als weitere bevorzugte "den TAT-Rezeptor hemmende Verbindung" ist ein TAT-Analogon zu nennen. Ein solches bindet nach wie vor an den TAT-Rezeptor, induziert jedoch nicht mehr den intrazellulären TAT-Rezeptor-Signalweg. Die Herstellung eines solchen Analogons erfolgt in üblicher Weise, wobei der Fachmann z.B. von dem in Arya, S., K., et al., Science, Band 229 (1985), Seiten 69-73 beschriebenen TAT ausgeht.

Der Ausdruck "eine TAT-hemmende bzw. abfangende Verbindung" umfaßt jegliche zur Hemmung bzw. zum Abfangen von TAT geeignete Verbindung. Vorzugsweise ist dies eine der folgenden Verbindungen:
- ein anti-TAT-Antikörper,
- ein TAT-Rezeptor,
- eine extrazelluläre TAT-Rezeptor-Domäne,
- eine Verbindung mit mindestens einer extrazellulären TAT-Rezeptor-Domäne und einem Träger,
- ein eine TAT-Bindungsstelle aufweisendes Peptid,
- eine Verbindung mit mindestens einem eine TAT-Bindungsstelle aufweisenden Peptid und einem Träger,
- eine TAT-Bindungsstelle auf Nukleinsäurebasis, und
- eine transdominante TAT-Mutante.

Die Herstellung einer solchen Verbindung erfolgt in üblicher Weise. Der Fachmann wird zur Herstellung eines anti-TAT-Antikörpers z.B. von dem in Arya, S., K. et al., supra beschriebenen TAT ausgehen. Er wird darauf achten, daß der Fc-Teil des Antikörpers in einem Individuum nicht als fremd angesehen wird. Verfahren, die das ermöglichen, sind ihm bekannt. Ferner wird er hinsichtlich einer TAT-Bindungsstelle auf Nukleinsäurebasis z.B. von der in Feng, S. und Holland, E., C., Nature, Band 334 (1988), Seiten 165-167 beschriebenen TAT-LTR-Sequenz ausgehen. Desweiteren wird der Fachmann bezüglich einer transdominanten TAT-Mutante z.B. von der in Echetebu, C., O. und Rice, A., P., J. Acquir. Immune Def. Syndrome, Band 6, (1993), Seiten 550-557 beschriebenen Mutante ausgehen.

Der Ausdruck "eine den intrazellulären TAT-Rezeptor-Signalweg hemmende Verbindung" umfaßt jegliche zur Hemmung des intrazellulären TAT-Rezeptor-Signalwegs geeignete Verbindung.

Der Ausdruck "eine den CD4-Rezeptor hemmende Verbindung" umfaßt jegliche zur Hemmung des CD4-Rezeptors geeignete Verbindung. Vorzugsweise ist dies ein anti-CD4-Rezeptor-Antikörper. Die Herstellung eines solchen erfolgt in üblicher Weise, wobei der Fachmann z.B. von dem in Capon, D., J. und Ward, R., H., R., Annu. Rev. Immunol. 9, (1991), Seiten 649-678, beschriebenen CD4-Rezeptor ausgeht. Der Fachmann wird darauf achten, daß der Fc-Teil des Antikörpers in einem Individuum nicht als fremd angesehen wird. Verfahren, die das ermöglichen, sind dem Fachmann bekannt.

Als weitere bevorzugte "den CD4-Rezeptor hemmende Verbindung" ist ein gp 120-Analogon zu nennen. Ein solches bindet nach wie vor an den CD4-Rezeptor, induziert jedoch nicht mehr den intrazellulären CD4-Rezeptor-Signalweg. Die Herstellung eines solchen Analogons erfolgt in üblicher Weise, wobei der Fachmann z.B. von dem in Capon, D., J. und Ward, R., H., R., supra beschriebenen gp 120 ausgeht.

Der Ausdruck "eine gp 120 hemmende bzw. abfangende Verbindung" umfaßt jegliche zur Hemmung bzw. Abfangung von gp 120 geeignete Verbindung. Vorzugsweise ist dies eine der folgenden Verbindungen:
- ein anti-gp 120-Antikörper,
- ein CD4-Rezeptor,
- eine extrazelluläre CD4-Rezeptor-Domäne,
- eine Verbindung mit mindestens einer extrazellulären CD4-Rezeptor-Domäne und einem Träger,
- ein eine gp 120-Bindungsstelle aufweisendes Peptid, und
- eine Verbindung mit mindestens einem eine gp 120-Bindungsstelle aufweisenden Peptid und einem Träger.

Die Herstellung einer solchen Verbindung erfolgt in üblicher Weise. Der Fachmann wird zur Herstellung eines anti-gp 120-Antikörpers z.B. von dem in Capon, D.J. und Ward, R., H., R., supra beschriebenen gp 120 ausgehen. Er wird darauf achten, daß der Fc-Teil des Antikörpers in einem Individuum nicht als fremd angesehen wird. Verfahren, die das ermöglichen, sind ihm bekannt. Ferner wird der Fachmann hinsichtlich der Herstellung eines CD4-Rezeptors bzw. einer extrazellulären CD4-Rezeptor-Domäne z.B. von dem in Capon, D., J. und Ward, R., H., R., supra beschriebenen CD4-Rezeptor bzw. seiner extrazellulären Domäne ausgehen. Desweiteren wird er zur Herstellung eines eine gp 120-Bindungsstelle aufweisenden Peptids z.B. die Kombination aus vorstehendem gp120 und vorstehendem CD4-Rezeptor bzw. seiner extrazellulären Domäne nutzen.

Die Herstellung einer Verbindung mit mindestens einer extrazellulären CD4-Rezeptor-Domäne bzw. einem mindestens eine gp 120-Bindungsstelle aufweisenden Peptid und einem Träger kann in analoger Weise erfolgen, wie nachstehend für eine Verbindung mit mindestens einer extrazellulären APO-1-Domäne und einem Träger beschrieben.

Der Ausdruck "eine den intrazellulären CD4-Rezeptor-Signalweg hemmende Verbindung" umfaßt jegliche zur Hemmung des intrazellulären CD4-Rezeptor-Signatwegs geeignete Verbindung.

Es wird verstanden, daß ein vorstehendes Mittel ein bis mehrere Verbindungen einer einzelnen Komponente aufweisen kann.

Erfindungsgemäß wird auch eine Verbindung mit mindestens einer extrazellulären APO-1-Domäne und einem Träger bereitgestellt, wobei die Domäne(n) und der Träger in einem Individuum nicht als fremd angesehen werden.

Der Ausdruck "Träger" umfaßt jegliche Verbindung, an die ein oder mehrere extrazelluläre APO-1 Domänen gebunden sein können.

In bevorzugter Ausführungsform ist der Träger ein Protein, z.B. Serumalbumin, Hämoglobin, Fibrinogen, Kollagen oder ein Fc-Teil eines Antikörpers, wobei letzteres bevorzugt ist. In ganz bevorzugter Ausführungsform ist die erfindungsgemäße Verbindung ein Fusionsprotein.

Eine erfindungsgemäße Verbindung kann in üblicher Weise hergestellt werden. Im Falle eines Fusionsproteins erweist sich das folgende Herstellungsverfahren als günstig:
(a) Amplifikation einer DNA durch übliche PCR-Technik, wobei die DNA für mindestens eine extrazelluläre APO-1-Domäne und eine am 3'-Ende dieser angebrachte Bindungsregion codiert,
(b) Amplifikation einer DNA durch übliche PCR-Technik, wobei die DNA für einen Protein-Träger und eine am 5'-Ende des Protein-Trägers angebrachte Bindungsregion codiert,
(c) Vereinigung der amplifizierten DNAs von (a) und (b) und weitere gemeinsame Amplifikation dieser durch übliche PCR-Technik, wobei ein dem 5'-Ende der DNA der APO-1-Domäne entsprechender Primer und ein dem 3'-Ende der DNA des Protein-Trägers entsprechender Primer verwendet werden, wodurch ein amplifiziertes, beide DNAs in **Fusion enthaltendes DNA-Fragment erhalten wird, und**
**(d) Insertion des DNA-Fragments von (c) in einen Expressionsvektor und Expression des DNA-Fragments in üblicher Weise.**

In bevorzugter Ausführungsform ist die Bindungsregion von (a) und (b) eine Antikörper-Hinge-Region oder ein Teil davon. Ferner kann sie auch eine Thrombinspaltstelle sein.

In vorstehendem Herstellungsverfahren werden verschiedene DNAs durch übliche PCR-Technik amplifiziert. Als DNA, die für mindestens eine extrazelluläre APO-1-Domäne codiert, wird der Fachmann z.B. die in der EP-92 107 060.3, supra beschriebene DNA als Basis verwenden. Dieser wird er am 3'Ende eine für eine Bindungsregion codierende DNA anfügen. Im Falle einer für eine Hinge-Region eines humanen Antikörpers oder einen Teil davon codierenden DNA wird der Fachmann z.B. auf die in Dübel, S., et al., Methods in Molecular and Cellular Biology, Band 3 (1992), Seiten 47-52 beschriebene DNA zurückgreifen. Hinsichtlich der für den Protein-Träger, z.B. Fc-Teil eines humanen Antikörpers, codierenden DNA wird der Fachmann z.B. ebenso auf die in Dübel, S. et al., supra beschriebene DNA zurückgreifen.

Die Expression des amplifizierten DNA-Fragments erfolgt in üblichen Vektoren, wie pCDN83, pCEV4 und pCDM8 zur Expression in tierischen Zellen, pGEMEX und pUC zur Expression in E. coli., sowie pY100 und YCpAD1 zur Expression in Hefe. Als tierische Zellen eignen sich insbesondere L-, COS- und CHO-Zellen, während als prokaryotische Mikroorganismen insbesondere E. coli-Stämme und als Hefezellen besonders solche von Saccharomyces und Pichia pastoris zu nennen sind.

Eine erfindungsgemäße Verbindung eignet sich bestens zur Hemmung von Apoptose. Sie kann hierzu allein oder in Kombination mit einer bis allen Komponenten verwendet werden, von:
(a) eine APO-1 hemmende Verbindung,
(b) eine weitere den APO-1-Ligand hemmende bzw. abfangende Verbindung,
(c) eine den intrazellulären APO-1-Signalweg hemmende Verbindung, wobei die Komponente(n) in einem Individuum nicht als fremd angesehen wird(werden).

Es wird auf vorstehende Ausführungen bezüglich der einzelnen Komponenten (a), (b) und (c) verwiesen. Diese Ausführungen gelten hier entsprechend.

Eine erfindungsgemäße Verbindung eignet sich insbesondere zur Hemmung von Apoptose bei einer mit einer HIV-Infektion assoziierten Erkrankung. Die erfindungsgemäße Verbindung kann hierzu allein oder in Kombination mit einer bis allen Komponenten verwendet werden, von:
(a) eine APO-1 hemmende Verbindung,
(b) eine weitere den APO-1-Ligand hemmende bzw. abfangende Verbindung,
(c) eine den intrazellulären APO-1-Signalweg hemmende Verbindung,
(d) eine den TAT-Rezeptor hemmende Verbindung,
(e) eine TAT-hemmende bzw. abfangende Verbindung,
(f) eine den intrazellulären TAT-Rezeptor-Signalweg hemmende Verbindung,
(g) eine den CD4-Rezeptor hemmende Verbindung,
(h) eine gp120 hemmende bzw. abfangende Verbindung, und
(i) eine den intrazellulären CD4-Rezeptor-Signalweg hemmende Verbindung, wobei die Komponente(n) in einem Individuum nicht als fremd angesehen wird(werden).

Es wird auf vorstehende Ausführungen bezüglich der einzelnen Komponenten (a) - (i) verwiesen, wobei die Komponenten (d) - (i) vorstehend mit (a) - (f) bezeichnet sind. Die vorstehenden Ausführungen gelten hier entsprechend.

Die vorliegende Erfindung eröffnet einen neuen Weg, Erkrankungen zu therapieren, bei denen Apoptose spezieller Zellen eine wichtige Rolle spielt. Insbesondere für die Therapie von AIDS stellt die vorliegende Erfindung einen Durchbruch dar.

Kurze Beschreibung der Zeichnung:
- Fig. 1: zeigt die Hemmung von Apoptose durch hu APO-1-lg bzw. anti-APO-1 F(ab')₂,
- Fig. 2a: zeigt die Stimulierung der proteolytischen Aktivität von ICE durch einen anti-APO-1-Antikörper,
- Fig. 2b: zeigt die Hemmung von Apoptose durch DCI,
- Fig. 2c: zeigt die Hemmung von Apoptose durch YVAD-CHO, und
- Fig. 2d: zeigt die Hemmung von Apoptose durch anti-Sinn ICE- bzw. CrmA-cDNA.

Die nachstehenden Beispiele erläutern die Erfindung.

### Beispiel 1: Herstellung eines Fusionsproteins, in dem eine extrazelluläre APO-1-Domäne über eine Antikörper-Hinge-Region an einen Fc-Teil eines humanen Antikörpers fusioniert ist.

Zur Herstellung vorstehenden Fusionsproteins wurden eine eine extrazelluläre APO-1-Domäne codierende cDNA (vgl. Oehm, A. et al., supra) und eine einen Fc-Teil eines humanen Antikörpers codierende cDNA (vgl. Dübel, S. et al., supra) einer üblichen PCR-Amplifikation unterzogen.

Als Primer für die cDNA der extrazellulären APO-1-Domäne wurden verwendet: 5' GCG AAG CTT GCC ACC ATG GTG GGC ATC TGG ACC CTC 3', wodurch eine Hind III-Stelle upstream einer vollständigen das Initiator-Methionin umgebenden Kozak-Consensus-Region eingeführt wird, und 5' GAC ACA ACA TTT GCG CTC GTT AGA TCT GGA TCC TTC 3', der für das 3'-Ende der extrazellulären APO-1-Domäne und die ersten 18 bp der Hinge-Region codiert.

Als Primer für die cDNA des Fc-Teils wurden verwendet:
5' GAG CGC AAA TGT TGT GTC GAG TGC 3', der für die ersten 18 bp der Hinge-Region und das 5'-Ende des Fc-Teils codiert, und 5' ATT AAG CAT TCT AGA TCA TTT ACC CGG AGA CAG GGA 3', der für das 3'-Ende des Fc-Teils codiert und eine Xbal-Stelle downstream des Stopcodons einführt.

Die erhaltenen PCR-Produkte wurden in einem "low melting point"-Agarosegel aufgetrennt und die Gelstückchen, die DNA-Moleküle richtiger Größe enthielten, wurden vereinigt. Mit einer Probe davon wurde eine weitere PCR-Amplifikation durchgeführt. Als Primer wurden nur jene vorstehenden verwendet, die dem 5'-Ende der extrazellulären APO-1-Domäne bzw. dem 3'-Ende des Fc-Teils entsprachen. Damit war es möglich, die extrazelluläre APO-1-Domäne über die gemeinsame Hinge-Region an den Fc-Teil zu fusieren. Es wurde ein "Fusions"-DNA-Fragment erhalten.

Dieses Fragment wurde mit HindIII und Xbal gespalten, über ein Agarosegel gereinigt und in dem Vektor pCDM8 kloniert. Die Sequenz des Inserts von pCDM8 wurde durch Dideoxynukleotid-Sequenzierung bestimmt.

### Beispiel 2: Hemmung von Apoptose durch hu APO-1-lg bzw. anti-APO-1F(ab')₂

SKW 6.4 Zellen (vgl. Oehm, A. et al., supra) sind Apoptose-positive Zellen, d.h. bei ihnen kann Apoptose induziert werden, z.B. durch Bindung eines anti-APO-1-Antikörpers.

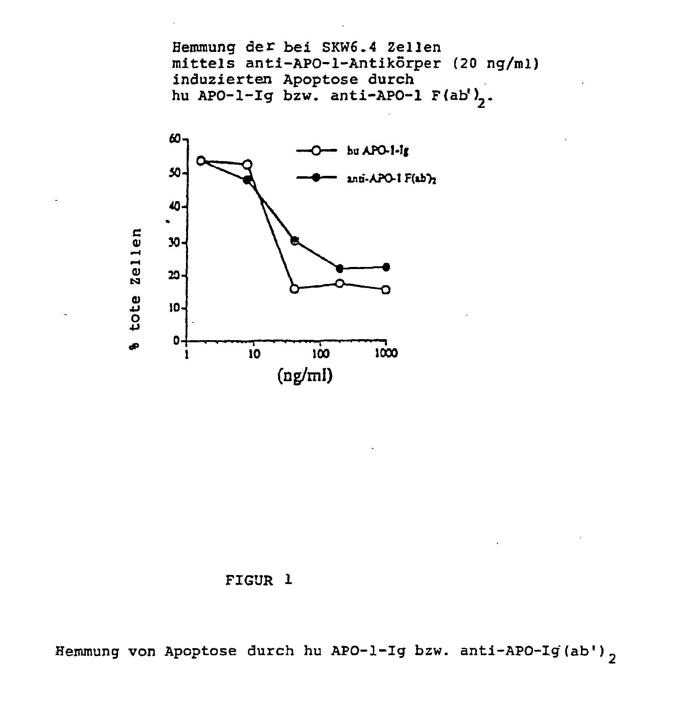

SKW 6.4 Zellen wurden 24 h mit einem anti-APO-1-Antikörper in Gegenwart verschiedener Mengen des Fusionsproteins von Beispiel 1 (hu APO-1-lg) bzw. von anti-APO-1-F(ab)₂ (vgl. vorstehend) inkubiert. Die Hemmung der Apoptose wurde bestimmt (vgl. Figur 1).

Es zeigte sich, daß das Fusionsprotein von Beispiel 1 und anti-APO-1-F(ab)₂ eine starke Apoptose-Hemmungs-Wirkung aufweisen. Diese ist bei dem Fusionsprotein besonders stark.

### Beispiel 3: Hemmung von Apoptose durch DCI, YVAD-CHO, Anti-Sinn ICE- bzw. CrmA-cDNA

L 929-APO-1 Zellen (vgl. Schulze-Osthoff, K. et al., EMBO J. 13, 4587-459, (1994)) sind wie SKW 6.4 Zellen von Beispiel 2 Apoptose-positiv.

### (a) Stimulierung der proteolytischen Aktivität von ICE durch einen anti-APO-1-Antikörper

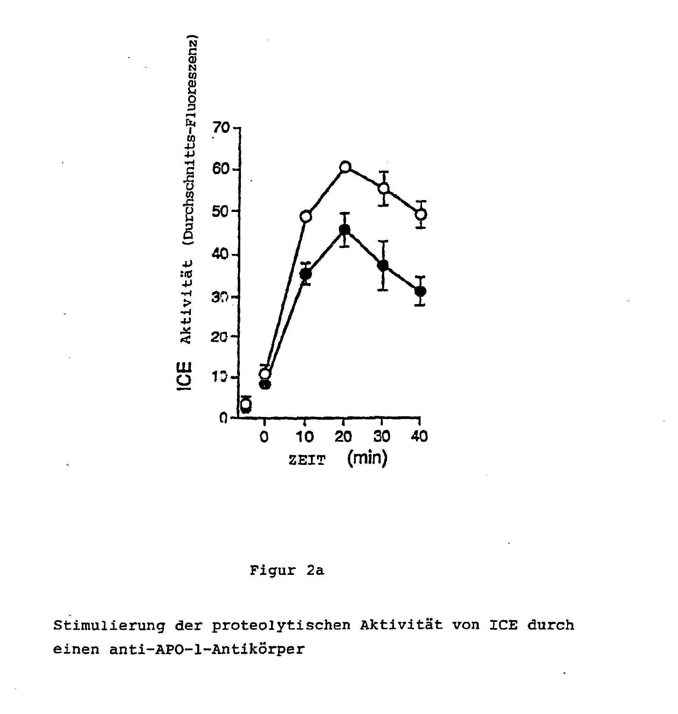

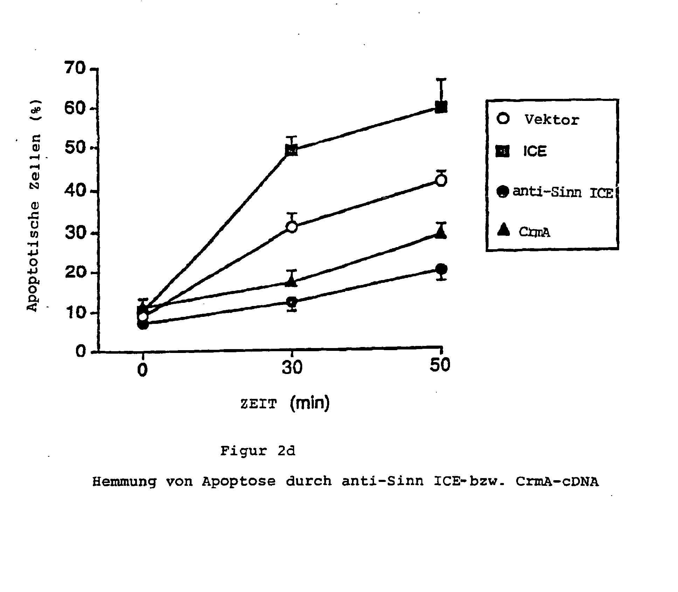

L 929-APO-1 Zellen ( o ) und SKW 6.4 Zellen (•) wurden kultiviert und mit anti-APO-1-Antikörper (1µg/ml) für die in Fig. 2a angegebenen Zeiten behandelt. 10 Minuten bevor die Zellen geerntet wurden, wurden sie mit 0,05 % Digitonin permeabilisiert und mit 20 µM des fluorogenen ICE-Substrats DABCYL-YVADAP-EDANS (Bachem, Bubendorf, Schweiz) inkubiert. Die Zellen wurden geerntet und durch eine FACS-Analyse unter Verwendung einer Anregungswellenlänge von 360 nm und einer Emissionsweilenlänge von 488 nm analysiert.

Es zeigte sich, daß die proteolytische Aktivität von ICE durch einen anti-APO-1-Antikörper stimuliert werden kann.

### (b) Hemmung einer anti-APO-1-Antikörper induzierten Apoptose durch DCI

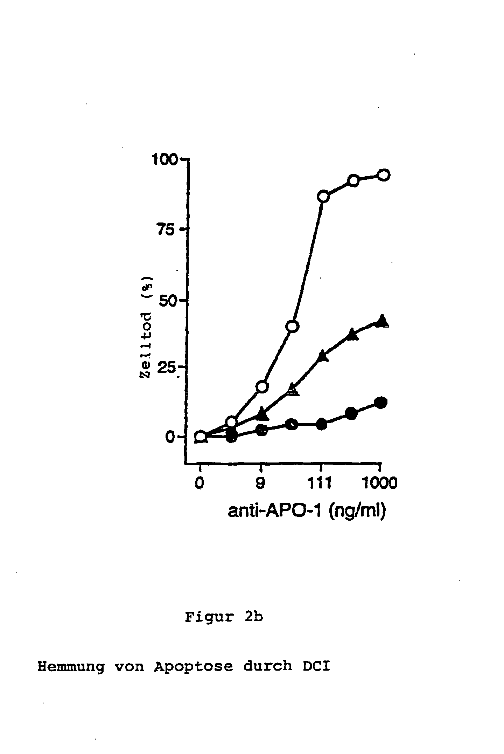

L 29-APO-1 Zellen wurden mit DCI (• 45µM, ▲ 15 µM) oder ohne DCI ( o ) inkubiert. Nach einer Stunde wurde anti-APO-1 Antikörper in den in Fig. 2b angegebenen Mengen zugegeben und 7 Stunden auf den Zellen gelassen. Der prozentuale Zelltod wurde durch Formazan-Produktion aus Diphenyltetrazoliumsalz (MTT-Assay) bestimmt.

Es zeigte sich, daß eine anti-APO-1-Antikörper induzierte Apoptose durch DCI gehemmt werden kann.

### (c) Hemmung einer anti-APO-1-Antikörper induzierten Apoptose durch YVAD-CHO

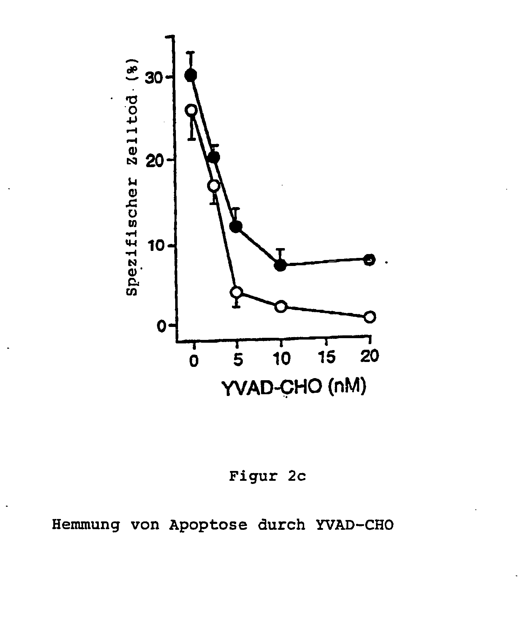

L 29-APO-1 Zellen (o) und SKW6.4 Zellen (•) wurden durch einen kurzen hypothonischen Schock permeabilisiert und mit den in Fig. 2c angegebenen Konzentrationen von YVAD-CHO 30 Minuten inkubiert. Die Zellen wurden mit anti-APO-1 Antikörper (1µg/ml) 60 Minuten inkubiert. Apoptose wurde bestimmt, indem eine DNA-Fragmentation mit dem Höchst-Farbstoff 33342 (Molecular Probes, Eugene, OR) gemessen wurde. Daten spezifischen Zelltods (Zelltod in Gegenwart eines anti-APO-1 Antikörpers minus Zelltod in Abwesenheit eines solchen Antikörpers) wurden aus Dreifach-Messungen erhalten, indem ein FACS Vantage-Flußzytometer verwendet wurde. Spontaner Zelltod in Abwesenheit eines anti-APO-1 Antikörpers betrug weniger als 7 %.

Es zeigte sich, daß eine anti-APO-1-Antikörper induzierte Apoptose durch YVAD-CHO gehemmt werden kann.

### (d) Hemmung einer anti-APO-1-Antikörper induzierten Apoptose durch anti-Sinn ICE- bzw. CrmA-cDNA

Folgende Expressionsplasmide wurden hergestellt:
**pCAGGS-ICE**

Eine Maus-ICE cDNA wurde durch RT-PCR isoliert, indem EL-4/c mRNA und oligo dT-Primer für die erste Strangsynthese verwendet wurden. Durch das Primerpaar 5'-ATCGGATCCAGCATGGCTGACAAGATCCTGAGG-3' und 5'-CGGCCTCGAGCATCATCTAAGGAAGTATTGGC-3' wurde ein 1322 bp PCR-Produkt erhalten, das als Probe zum Screening einer in pCAGGS (vgl. Niwa, H. et al., Gene 108, 193 - 200 (1994)) klonierten E14/13 cDNA Expressions-Genbank verwendet wurde. Es wurde ein 1387 bp ICE cDNA Klon erhalten, dessen Sequenz durch DNA-Sequenzierung bestimmt wurde. Dieser Klon wurde mit pCAGGS-ICE bezeichnet.
pCAGGS-anti-Sinn ICE

In pCAGGS wurde ein 320 bp EcoRI-Fragment der vorstehenden ICE cDNA in umgekehrter Orientierung kloniert, wobei das Fragment 48 bp der 5' UTR und die ersten 255 bp des ICE-ORF enthielt. Es wurde das Expressionsplasmid pCAGGS-anti-Sinn ICE erhalten.
pSV25S-CrmA

In einer üblichen PCR wurde durch Verwendung des Primerpaares 5'-GC-GAAGCTTACACGACCAATATCGATTACTA-3' und 5'-CGCCATGGTTAA-CAATTAGTTGTCGGAGAG-3'aus Vaccinia-Virus DNA eine für CrmA codierende cDNA erhalten. Diese cDNA wurde als HindIII/Kpnl-Fragment in das bekannte Plasmid pSV25S kloniert. Es wurde das Expressionsplasmid pSV25S-CrmA erhalten.

Die vorstehenden Expressionsplasmide wurden zur Transfektion von L 929-APO-1 Zellen verwendet. Hierzu wurden die Zellen in TBS-Puffer aufgenommen und 10 Minuten auf Eis äquilibriert. Die Zellen wurden jeweils mit 20 µg vorstehender Expressionsplasmide transfiziert, indem ein Biorad-Elektroporator (960 µFD, 220 V) verwendet wurde. Nach der Elektroporation wurden die Zellen weitere 30 Minuten auf Eis gehalten, bevor sie in Zellkulturplatten ausgesät wurden. Tote Zellen wurden nach 16 Stunden durch einen Waschschritt entfernt. Lebende Zellen wurden mit anti-APO-1-Antikörper (1µg/ml) für die in Fig. 2d angegebenen Zeiten behandelt. Apoptose wurde, wie vorstehend beschrieben, durch FACS-Analyse bestimmt, wobei der Hoechst-Farbstoff 33342 verwendet wurde. Daten wurden als prozentualer Zelltod aus Zweifach-Experimenten ermittelt.

Es zeigte sich, daß eine anti-APO-1-Antikörper induzierte Apoptose durch anti-Sinn ICE bzw. CrmA gehemmt werden kann.

## Patentansprüche

1. Fusionsprotein, umfassend eine extrazelluläre Domäne von humanem APO-1 und einen Träger.

2. Fusionsprotein nach Anspruch 1,
**dadurch gekennzeichnet,**
**dass** der Träger ein Protein ist.

3. Fusionsprotein nach einem der Ansprüche 1 bis 2,
**dadurch gekennzeichnet,**
**dass** das Protein ein Fc-Teil eines Antikörpers ist.

4. Fusionsprotein nach Anspruch 3,
**dadurch gekennzeichnet,**
**dass** das Protein der Fc-Teil eines humanen Antikörpers ist.

5. Fusionsprotein nach Anspruch 3 oder 4,
**dadurch gekennzeichnet,**
**dass** die extrazelluläre humane APO-1 Domäne über eine Antikörper-Hinge-Region an den Fc-Teil des Antikörpers fusioniert ist.

6. Fusionsprotein nach einem der Ansprüche 1 bis 9,
**dadurch gekennzeichnet,**
**dass** die extrazelluläre humane APO-1 Domäne bis zu Aminosäure 171 des humanen APO-1 Moleküls reicht.

7. DNA-Molekül,
**dadurch gekennzeichnet,**
**dass** es für ein Fusionsprotein nach einem der Ansprüche 1 bis 6 kodiert.

8. DNA-Molekül nach Anspruch 7, das für ein Fusionsprotein kodiert, bei dem eine extrazelluläre APO-1 Domäne über eine Antikörper-Hinge-Region an einen Fc-Teil eines humanen Antikörpers fusioniert ist, wobei die APO-1 Domäne aus den Aminosäuren 1-171 des humanen APO-1 Moleküls besteht.

9. Verfahren zur Herstellung eines Fusionsproteins nach einem der Ansprüche 1 bis 6, umfassend die folgenden Verfahrensschritte:
(a) Amplifikation einer DNA durch übliche PCR-Technik, wobei die DNA für mindestens eine extrazelluläre APO-1 Domäne und eine am 3'-Ende dieser angebrachte Bindungsregion kodiert,
(b) Amplifikation einer DNA durch übliche PCR-Technik, wobei die DNA für einen Protein-Träger und eine am 5'-Ende des Protein-Trägers angebrachte Bindungsregion kodiert,
(c) Vereinigung der amplifizierten DNAs von (a) und (b) und weitere gemeinsame Amplifikation dieser durch übliche PCR-Technik, wobei am 5'-Ende der DNA der APO-1 Domäne entsprechender Primer und ein dem 3'-Ende der DNA des Protein-Trägers entsprechender Primer verwendet werden, wodurch ein amplifiziertes, beide DNAs in Fusion enthaltendes DNA-Fragment erhalten wird, und
(d) Insertion des DNA-Fragments von (c) in einen Expressionsvektor und Expression des DNA-Fragments in üblicher Weise.

10. Arzneimittel,
**dadurch gekennzeichnet,**
**dass** es als Wirkstoff ein Fusionsprotein nach einem der Ansprüche 1 bis 6 oder ein DNA-Molekül nach Anspruch 7 oder 8 enthält.

11. Verwendung einer einen APO-1-Ligand hemmenden bzw. abfangenden Verbindung zur Herstellung eines Arzneimittels für die Behandlung einer Erkrankung, bei der Apoptose auftritt.

12. Verwendung nach Anspruch 11,
**dadurch gekennzeichnet,**
**dass** die Erkrankung AIDS ist.

13. Verwendung nach Anspruch 11,
**dadurch gekennzeichnet,**
**dass** die Erkrankung eine Autoimmunerkrankung ist.

14. Verwendung nach einem der Ansprüche 11 bis 13,
**dadurch gekennzeichnet,**
**dass** das Arzneimittel ein Fusionsprotein nach einem der Anspruche 1 bis 6 oder ein DNA-Molekül nach Anspruch 7 oder 8 enthält.

15. Verwendung nach Anspruch 14,
**dadurch gekennzeichnet,**
**dass** das Arzneimittel einen anti-APO-1-Ligand-Antikörper enthält.
